# EUROPEAN PATENT SPECIFICATION

(11) **EP 1 106 613 B1**
(45) Date of publication and mention of the grant of the patent: **10.04.2002**
(21) Application number: 99309834.2
(22) Date of filing: 07.12.1999
(51) Int. Cl.: C07D 403/14, C09J 175/04

(54) **Uretedione derivative, curable resin composition comprising the same, and process for producing the same**
Uretedionderivat, es enthaltende härtbare Harzzusammensetzung und Verfahren zu seiner Herstellung
Dérivé d'urétédione, composition de résine durcissable le contenant et son procédé de fabrication

(43) Date of publication of application: 13.06.2001
(73) Proprietor: Chinese Petroleum Corporation, Taipei City (TW)
(72) Inventor: Ling, Hao-Jan, Lo-Tung Chen, I-Lan Hsien (TW); Chen, Kan-Nan, Taipei City (TW); Lai, Jian-Zei, Chia-Yi City (TW); Lin, Yun-Shan, Tamsui 251 (TW)
(74) Representative: Allard, Susan Joyce

(56) References cited:
- EP-A- 0 781 771
- GB-A- 1 213 640
- US-A- 2 302 288
- US-A- 2 390 165
- CHEMICAL ABSTRACTS, vol. 129, no. 24, 14 December 1998 (1998-12-14) Columbus, Ohio, US; abstract no. 317044, NAKAMURA N. ET AL: "Curable resin compositions" XP002137075 & JP 10 265681 A (TOYO INK MFG. CO., LTD.) 6 October 1998 (1998-10-06)
- CHEMICAL ABSTRACTS, vol. 103, no. 4, 29 July 1985 (1985-07-29) Columbus, Ohio, US; abstract no. 23836, MITSUBISHI RAYON CO., LTD.: "Radiation-curable resin compositions" XP002137076 & JP 60 032822 A (MITSUBISHI RAYON CO. LTD.) 20 February 1985 (1985-02-20)

## Description

The present invention relates to a uretedione derivative, more particularly to a uretedione derivative for aqueous-based polyurethane.

Polyurethanes are a type of polymers that can have widely differing properties. Such versatility and multitude properties permit some of the polyurethanes to exhibit an elasticity and a flexibility similar to those of rubbers, and others to exhibit a mechanical strength and a hardness similar to those of plastics. As such, polyurethanes are wildly used in the manufacture of products, such as shoesoles, synthetic leathers, adhesives, sealants, printing inks, foams, films, coatings, and fiber modifiers.

Applications of solvent-based polyurethanes and technologies for the process of producing the same are well developed. However, due to problems with respect to the environment, economy, sanitation, and safety, the solvent-based polyurethane tends to be gradually replaced by aqueous-based polyurethane which is an environmental friendly product that dispenses with the use of the solvent.

While there has been a great demand for aqueous-based polyurethane, the physical and mechanical properties of the same are still insufficient in comparison with those of the solvent-based polyurethane. Difficulties have been encountered for current technologies for producing aqueous-based polyurethane in enhancing the molecular weight and the cross-linking density of the polyurethane. Such properties are often improved by post-curing reaction which unfortunately results in limiting the applications of the aqueous-based polyurethane.

There are several types of self-emulsifiable aqueous-based polyurethane available in the market. These aqueous-based polyurethanes can be classified into non-ionic, cationic, and anionic aqueous-based polyurethanes depending on the nature of the hydrophilic group of the polyurethane. For instance, when dissolved in water, the carboxy groups of the anionic aqueous-based polyurethane provide surface charges to the surrounding of the polyurethane molecules (particles), thereby causing repulsion between the polyurethane molecules (particles) and resulting in uniform distribution of the polyurethane molecules in the water phase. Such behavior permits the aqueous-based polyurethane to form into a polyurethane emulsion upon mixing with water, and is similar to a surfactant which acts as an emulsifier and which is formed into a plurality of micro-cells in a water phase. Because of the hydrophilic property of the carboxy group, the polyurethane becomes self-emulsifiable or water-reducible in the water phase . Such aqueous-based polyurethanes have a common disadvantage similar to that of a polymeric surfactant in that, after drying into a film, such film exhibits a high water absorptivity.

As described in the publications, the improvements on aqueous-based polyurethane are normally performed by post-curing reaction to enhance the molecular weight and the cross-linking density of the polyurethane so as to broaden the applications of the same. Conventionally, such post-curing reaction is carried out by mixing "two components" together, i.e., adding a liquid containing the post-curing agent into another liquid containing the aqueous-based polyurethane before the application. However, such mixing of "two components" may result in instability to the quality of the polyurethane products due to the variations of the ratio of the "two components" and the degree of the uniformity of the agitation. The occurrence of such instability is particularly severe in a batch process, and can significantly restrict the applications of the aqueous-based polyurethane.

In the case of the process for producing anionic (carboxy group) polyurethane emulsion, the addition of water into an isocyanate-terminated urethane prepolymer is an essential step to form the polyurethane emulsion. The formation of the anionic carboxy group polyurethane emulsion is better described from the following scheme:

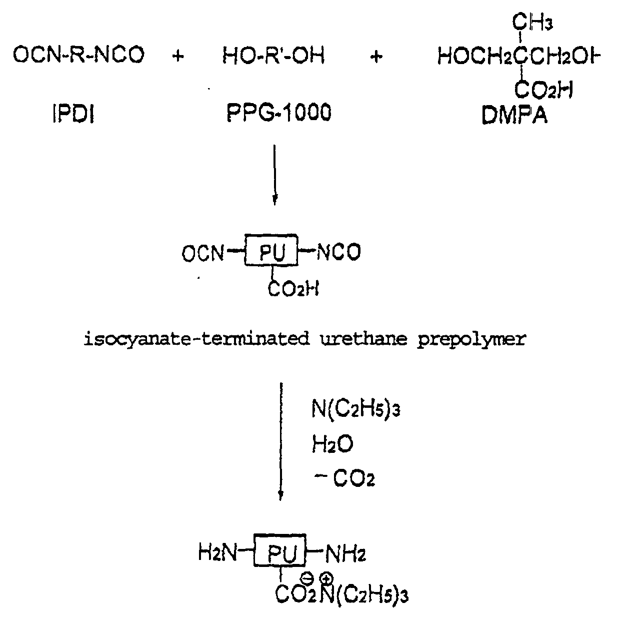

As described in the above reaction scheme, isocyanate-terminated urethane prepolymer is prepared by reacting isophorone diisocyanate with polypropylene glycol in the presence of dimethylpropionic acid. The terminal isocyanate groups of the thus formed urethane prepolymer are hydrolyzed to amino groups by water. The amino groups of the urethane prepolymer may result in a self-chain extending reaction by reacting with the isocyanate groups of the urethane prepolymer to form urea bonding, or may be present in the water phase without further reaction. While the presence of the amino groups and the carboxy groups of the aqueous-based polyurethane can stabilize the polyurethane emulsion, they also result in high water absorptivity which significantly affects the subsequent processing of the polyurethane emulsion, such as dyeing.

Therefore, it is an object of the present invention to provide a curing agent containing a uretedione derivative which is capable of overcoming the problems described above.

According to one aspect of the present invention, there is provided a uretedione derivative having the following formula (I) : 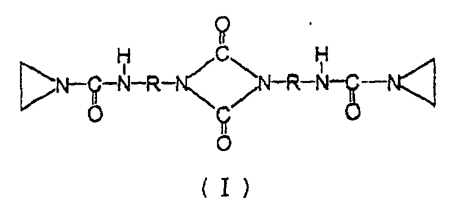 wherein R represents a unsubstituted or substituted C₁-C₂₀ hydrocarbyl group.

According to another aspect of the present invention, there is provided a curable resin composition comprising the uretedione derivative of formula (I) and a resin which is reactive toward the uretedione derivative and which comprises amino and carboxy groups.

According to yet another aspect of the present invention, there is provided a process for producing the uretedione derivative of formula (I), comprising the step of reacting a uretedione with an aziridine to form the uretedione derivative.

The above described instability and high water absorptivity for the aqueous-based polyurethane can be eliminated by introducing a curing agent discovered in the present invention into an isocyanate-terminated urethane prepolymer.

The curing agent of this invention contains a uretedione derivative which is prepared by addition reaction of a uretedione with an aziridine to form a compound containing uretedione and aziridine functional groups and having the following formula: 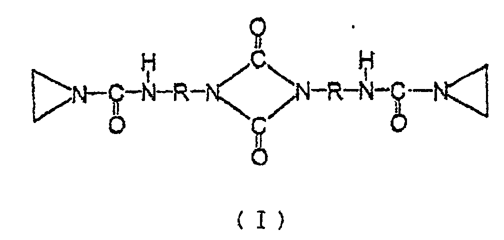 wherein R represents a unsubstituted or substituted C₁-C₂₀ hydrocarbyl group. The above reaction and the reaction for preparing the uretedione can be illustrated in the following scheme:

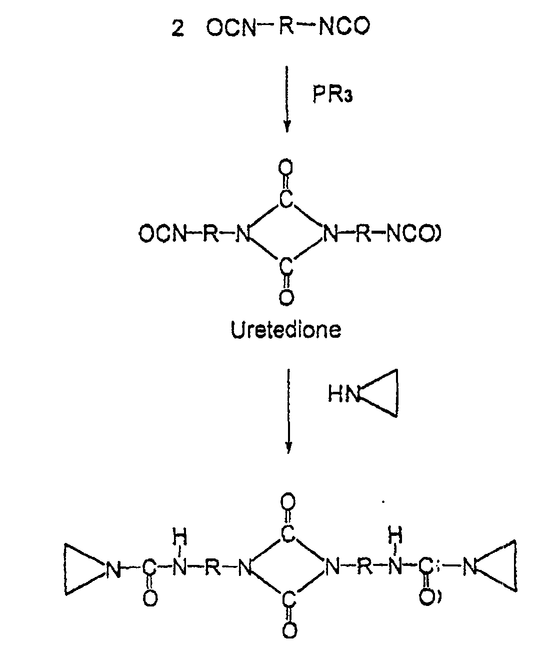

Uretediones can be prepared in the presence of a specific catalyst, such as triethylphosphines, through dimerization of aliphatic diisocyanates, such as isophorone diisocyanate (IPDI), hexamethylene diisocyanate(HDI), and hydrogenated methylene diphenyl 4,4'-diisocyanate (H₁₂MDI).

With the above described curing agent of this invention, the isocyanate-terminated urethane prepolymer can be transformed into a self-curable urethane prepolymer which will form into a "single component" self-curable polyurethane emulsion when mixed with water. The term "single component" used herein is simply to distinguish from the above described "two components", and that it can be understood that the polyurethane emulsion according to this invention is self-curable, thereby dispensing from the use of an additional liquid containing post-curing agent. The role of the curing agent of this invention can be better understood by the following description.

Before forming into a polyurethane emulsion, the isocyanate-terminated urethane prepolymer is simply mixed with the uretedione derivative of formula (I) without reacting with the latter. When further mixed with water to form the polyurethane emulsion, the terminal isocyanate groups of the isocyanate-terminated urethane prepolymer are first hydrolyzed into amino groups which then immediately undergo an addition reaction with the uretedione groups of the uretedione derivative contained in the above described mixture to form urea bonding, thereby resulting in a cross-linking reaction. Such cross-linking reaction is carried out through the ring-opening of the uretedione, and is illustrated by the following scheme:

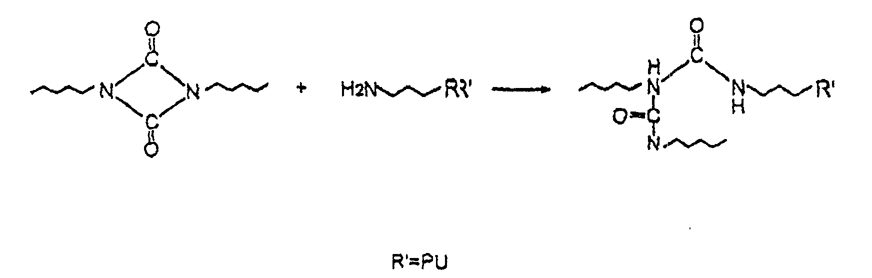

After the ring opening of the uretedione, the uretedione derivative originally contained in the mixture becomes part of the cross-liked polyurethane in the polyurethane emulsion, thereby introducing the aziridine groups therein. The polyurethane emulsion according to this invention normally has a pH value greater than 8. At such condition, the introduced aziridine groups are retained in a stable condition in the polyurethane emulsion. When the polyurethane emulsion is subjected to a drying operation, the pH value of the emulsion will decrease. When the pH value decreases to less than 6, the carboxy group of the cross-linked polyurethane will start reacting with the introduced aziridine groups, thereby resulting in another cross-linking reaction through the ring-opening of the aziridine. Such cross-linking reaction can be illustrated by the following scheme:

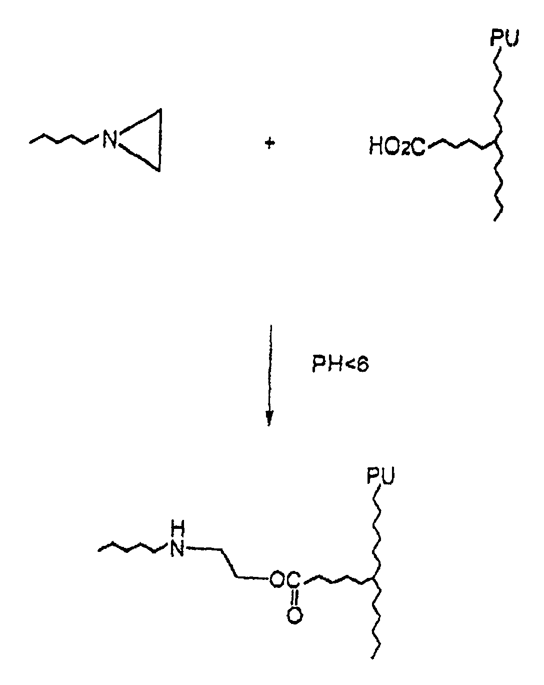

The invention will now be specifically described by the following examples which are not meant to limit the scope of this invention.

### Example 1

A round-bottom flask was charged with 20g of isophorone diisocyante (IPDI) and 0.5% by weight of triethyl phosphine, based on the total weight of the above two compounds. Dimerization of the isophorone diisocyante for forming the uretedione was carried out in a water bath at a temperature ranging from 85 to 90°C. The reaction was terminated when the isocyanate number reached a stoichiometric number which was half of the initial isocyanate number. Five grams of aziridine was added dropwise into the flask to react with the above formed uretedione to form the curing agent of this invention. The reaction was carried out at a temperature of 50°C for about 2 hours. The reaction was completed when the isocyanate number dropped to about zero (i.e., the absorption peak based on the NCO group was not observed at 2261cm⁻¹ in the infrared spectrum measurement). The thus formed curing agent exhibits two new absorption peaks which are observed at 1540 and 1668cm⁻¹ in the infrared spectrum measurement and which represent the uretedione and the aziridine functional groups of the curing agent, respectively.

### Example 2

Isophorone diisocyanate (IPDI), polypropylene glycol (PPG-1000), and dimethylolpropanic acid (DMPA) were used as the starting material for the preparation of the isocyanate-terminated urethane prepolymer. The ratio of IPDI:PPG-1000:DMPA was 4:2:1.
Reaction was carried out at a temperature ranging from 95 to 100°C for about 4 hours. The reaction was completed when the isocyanate number reached 3.5%, based on the initial number of the isocyanate number contained in the reactants. The thus formed NCO-terminated urethane prepolymer was then cooled to room temperature. 100g of the above prepolymer was mixed with 3.7g of the curing agent obtained from Example 1. The above formed mixture was neutralized with triethyl amine, and was diluted with acetone to obtain a workable viscosity (e.g. 1000 cps). The diluted mixture was then added with water to form the aqueous-based polyurethane emulsion. The polyurethane emulsion was subjected to drying to form a polyurethane film. The measured tensile strengths of the above formed polyurethane film were 2.2 kg/cm² with an elongation of 100%, 3.8 kg/cm² with an elongation of 200%, and 7.9kg/cm² with an elongation of 500%. With the same corresponding elongations described above, the measured tensile strengths of an aqueous-based polyurethane film which had not been cross-linked were 0.5, 1.0, and 1.9kg/cm², respectively. The polyurethane film formed in this example also exhibits a gel content of 94.7% and an ethanol-swollen of 377.5%. The aqueous-based polyurethane film which had not been cross-linked is soluble in solvent, such as tetrahydrofuran(THF) and ethanol.

### Example 3

A curing agent of this invention was prepared according to the same procedure as that: of Example 1 except that the IPDI was replaced by H₁₂MDI. A polyurethane film containing the above formed curing agent was formed by performing the same procedure as that of Example 2. The measured tensile strengths of the thus formed polyurethane film were 4.3 kg/cm² with an elongation of 100% and 7.0 kg/cm² with an elongation of 200%. The polyurethane film formed in this example also exhibits a gel content of 97.5% and an ethanol-swollen of 220%.

## Claims

1. A uretedione derivative having the following formula (I): 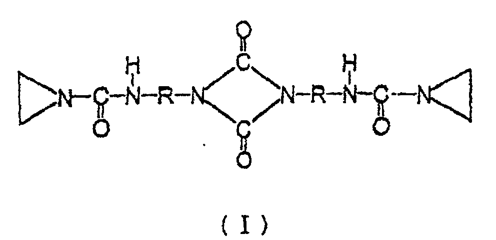 wherein R represents a unsubstituted or substituted C₁-C₂₀ hydrocarbyl group.

2. The uretedione derivative of Claim 1,
**characterized in that** said uretedione derivative is prepared by reacting a uretedione with an aziridine.

3. The uretedione derivative of Claim 2, further
**characterized in that** said uretedione is prepared by dimerizing a diisocynate.

4. The uretedione derivative of Claim 3, further
**characterized in that** said diisocynate is selected from the group consisting of isophorone diisocyanate, hexamethylene diisocyanate, and hydrogenated methylene diphenyl 4,4'-diisocyanate.

5. A curable resin composition, comprising a uretedione derivative of formula (I) of Claim 1 and a resin which is reactive toward said uretedione derivative and which comprises amino and carboxy groups.

6. The curable resin composition of Claim 5, **characterized in that** said uretedione derivative is prepared by reacting a uretedione with an aziridine.

7. The curable resin composition of Claim 6, further **characterized in that** said resin is an isocyanate-terminated urethane prepolymer having terminal isocyanate groups.

8. The curable resin composition of Claim 7, further comprising water which reacts with said isocyanate groups of said resin to form amino groups.

9. The curable resin composition of Claim 8, **characterized in that** said uretedione group of said uretedione derivative reacts with said amino groups of said resin at a pH value > 8 to form urea bonding.

10. The curable resin composition of Claim 9 , further **characterized in that** said urethane prepolymer comprises a carboxy group which reacts with said aziridine group of said uretedione derivative at a pH value < 6.

11. A process for producing a uretedione derivative of formula (I) of Claim 1, comprising the step of reacting a uretedione with an aziridine to form said uretedione derivative.

12. The method of Claim 11, **characterized in that** said uretedione is prepared by dimerizing a diisocynate.

13. The method of Claim 12, further **characterized in that** said diisocynate is selected from the group consisting of isophorone diisocyanate, hexamethylene diisocyanate, and hydrogenated methylene diphenyl 4,4'-diisocyanate.

## Patentansprüche

1. Uretedionderivat mit der nachstehenden Formel (I): 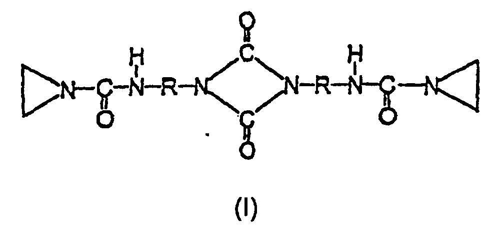 worin R eine unsubstituierte oder substituierte C₁-C₂₀ Kohlenwasserstoffgruppe darstellt.

2. Uretedionderivat nach Anspruch 1, **dadurch gekennzeichnet, daß** das Uretedionderivat durch Umsetzen eines Uretedions mit einem Aziridin hergestellt wird.

3. Uretedionderivat nach Anspruch 2, weiter **dadurch gekennzeichnet, daß** das Uretedionderivat durch Dimerisierung eines Diisocyanats hergestellt wird.

4. Uretedionderivat nach Anspruch 3, weiter **dadurch gekennzeichnet, daß** das Diisocyanat aus der Gruppe, bestehend aus Isophorondiisocyanat, Hexamethylendiisocyanat und hydriertem Methylendiphenyl-4,4'-diisocyanat ausgewählt ist.

5. Härtbare Harzzusammensetzung, umfassend ein Uretedionderivat nach Formel (I) von Anspruch 1, und ein mit dem Uretedionderivat reaktives, Amino- und Carboxygruppen umfassendes Harz.

6. Härtbare Harzzusammensetzung nach Anspruch 5, **dadurch gekennzeichnet, daß** das Uretedionderivat durch Umsetzen eines Uretedions mit einem Aziridin hergestellt wird.

7. Härtbare Harzzusammensetzung nach Anspruch 6, weiter **dadurch gekennzeichnet, daß** das Harz ein Isocyanat-terminiertes Urethanpräpolymer mit endständigen Isocyanatgruppen ist.

8. Härtbare Harzzusammensetzung nach Anspruch 7, weiter umfassend Wasser, das mit den Isocyanatgruppen des Harzes reagiert, wobei Aminogruppen gebildet werden.

9. Härtbare Harzzusammensetzung nach Anspruch 8, **dadurch gekennzeichnet, daß** die Uretediongruppen des Uretedionderivats bei einem pH-Wert> 8 mit den Aminogruppen des Harzes reagieren, wobei Hamstoffbindungen gebildet werden.

10. Härtbare Harzzusammensetzung nach Anspruch 9, weiter **dadurch gekennzeichnet, daß** das Urethanpräpolymer eine Carboxygruppe umfaßt, die bei einem pH-Wert < 6 mit der Aziridingruppe des Uretedionderivats reagiert.

11. Verfahren zur Herstellung eines Uretedionderivats nach Formel (I) von Anspruch 1, umfassend den Schritt des Umsetzens eines Uretedions mit einem Aziridin, wobei das Uretedionderivat gebildet wird.

12. Verfahren nach Anspruch 11, **dadurch gekennzeichnet, daß** das Uretedion durch Dimerisierung eines Diisocyanats hergestellt wird.

13. Verfahren nach Anspruch 12, weiter **dadurch gekennzeichnet, daß** das Diisocyanat aus der Gruppe, bestehend aus Isophorondiisocyanat, Hexamethylendiisocyanat und hydriertem Methylendiphenyl-4,4'-diisocyanat ausgewählt wird.

## Revendications

1. Dérivé d'urétedione répondant à la formule (I) suivante : 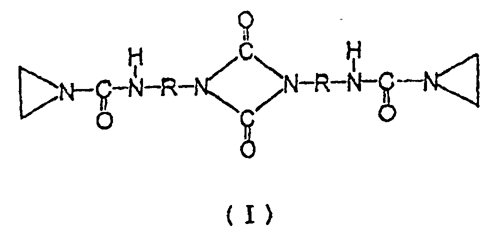 dans laquelle R représente un groupe hydrocarbyle en C₁ à C₂₀ non substitué ou substitué.

2. Dérivé d'urétedione suivant la revendication 1, **caractérisé en ce qu'**il est préparé en faisant réagir une urétedione avec une aziridine.

3. Dérivé d'urétedione suivant la revendication 2, **caractérisé en outre en ce que** ladite urétedione est préparée par dimérisation d'un diisocyanate.

4. Dérivé d'urétedione suivant la revendication 3, **caractérisé en outre en ce que** ledit diisocyanate est choisi dans le groupe consistant en l'isophorone-diisocyanate, l'hexaméthylène-diisocyanate et un méthylène-diphényl-4,4'-diisocyanate hydrogéné.

5. Composition de résine durcissable, comprenant un dérivé d'urétedione de formule (I) suivant la revendication 1 et une résine qui est réactive avec ledit dérivé d'urétedione et qui comprend des groupes amino et carboxy.

6. Composition de résine durcissable suivant la revendication 5, **caractérisée en ce que** le dérivé d'urétedione est préparé en faisant réagir une urétedione avec une aziridine.

7. Composition de résine durcissable suivant la revendication 6, **caractérisée en outre en ce qu'**elle consiste en un prépolymère d'uréthanne à terminaison isocyanate, ayant des groupes isocyanate terminaux.

8. Composition de résine durcissable suivant la revendication 7, comprenant en outre de l'eau qui réagit avec les groupes isocyanate de ladite résine pour former des groupes amino.

9. Composition de résine durcissable suivant la revendication 8, **caractérisée en ce que** le groupe urétedione du dérivé d'urétedione réagit avec les groupes amino de ladite résine à une valeur de pH supérieure à 8 pour former une liaison urée.

10. Composition de résine durcissable suivant la revendication 9, **caractérisée en outre en ce que** le prépolymère d'uréthanne comprend un groupe carboxy qui réagit avec le groupe aziridine du dérivé d'urétedione à une valeur de pH inférieure à 6.

11. Procédé pour la production d'un dérivé d'urétedione de formule (I) suivant la revendication 1, comprenant l'étape consistant à faire réagir une urétedione avec une aziridine pour former ledit dérivé d'urétedione.

12. Procédé suivant la revendication 11, **caractérisé en ce que** l'urétedione est préparée par dimérisation d'un diisocyanate.

13. Procédé suivant la revendication 12, **caractérisé en outre en ce que** le diisocyanate est choisi dans le groupe consistant en l'isophorone-diisocyanate, l'hexaméthylène-diisocyanate et un méthylène-diphényl-4,4'-diisocyanate hydrogéné.
